# EUROPEAN PATENT APPLICATION

(11) **EP 2 792 933 A1**
(43) Date of publication of application: **22.10.2014**
(21) Application number: 12857759.0
(22) Date of filing: 11.12.2012
(51) Int. Cl.: F21S 2/00, F21V 3/04, F21V 8/00, F21V 23/00, A61B 19/00, F21Y 101/00, F21Y 101/02

(54) **LIGHT SOURCE SYSTEM HAVING A PLURALITY OF LIGHT-CONDUCTING ROUTES**

(30) Priority: 13.12.2011 JP 2011272561
(71) Applicant: Olympus Corporation, Shibuya-ku Tokyo 151-0072 (JP)
(72) Inventor: ITO, Takeshi, Hachioji-shi Tokyo 192-8512 (JP); YAMAMOTO, Eiji, Hachioji-shi Tokyo 192-8512 (JP); NISHIO, Masahiro, Hachioji-shi Tokyo 192-8512 (JP)
(74) Representative: Winter, Brandl, Fürniss, Hübner, Röss, Kaiser, Polte - Partnerschaft mbB
(86) International application number: PCT/JP2012/082072
(87) International publication number: WO 2013/089102

(57) **Abstract**

In a light source system constituted by combining a light source module having a light source and a light source light emitting end, and an irradiation module having an optical connection terminal, a light guide member and a light emitting member and being mechanically detachably attachable to the light source module, when a first light guide route is defined as a route comprising a first light source, a first light source light emitting end (32), a first optical connection terminal (70), a first light guide member (76), and a first light emitting member (78), and when a second light guide route is defined as a route comprising a second light source, a second light source light emitting end (52), a second optical connection terminal (72), a second light guide member (82), and a second light emitting member (88), the first light source light emitting end and the first optical connection terminal are constituted based on desirable first optical specifications, and the second light source light emitting end and the second optical connection terminal are constituted based on desirable second optical specifications, so that the first or second light guide route can be constituted.

## Description

### Technical Field

The present invention relates to a light source system constituted by combining a light source module and an irradiation module, and more particularly, it relates to a light source system having light guide routes.

### Background Art

As one example of a light source system on which two types of light sources and two types of light guide members corresponding to these light sources are mounted, there is disclosed in, for example, Jpn. Pat. Appln. KOKAI Publication No. H10-337271, a fluoroendoscope in which a laser light source and a usual illuminating light source are combined. This fluoroendoscope is constituted so that usual illuminating light and laser light can be applied onto a living tissue, and the image light obtained during the irradiation with the usual illumination and fluorescence obtained by a TV camera during the irradiation with the laser light can be observed via an image bundle. More specifically, the laser light is guided to a tip of an endoscope by use of an optical fiber for laser transmission, and the usual illuminating light is guided to the tip of the endoscope by use of an optical fiber bundle for usual illumination. In the above Jpn. Pat. Appln. KOKAI Publication No. H10-337271, there is described a structure where bare fibers of the optical fiber bundle for usual illumination are wound around the optical fiber for laser transmission.

As seen in the above-mentioned constitution example of a fluoroendoscope, a light source system on which two types of light sources.and two types of light guide members corresponding to these light sources are mounted has the following problem. That is, exclusive and special light guide mechanisms corresponding to the respective light sources are required, and it is necessary to individually construct the exclusive light source system for each purpose (a usual illuminating light source system and a laser light source system for fluorescent observation in the case of the above Jpn. Pat. Appln. KOKAI Publication No. H10-337271). Therefore, the conventional light source system is expensive and has poor cost performance.

### Summary of Invention

The present invention has been developed in view of the above respect, and an object thereof is to provide a light source system capable of coping with various purposes without having to construct an individual exclusive system.

According to an aspect of the invention, there is provided a light source system constituted by combining:
a light source module, and
an irradiation module mechanically detachably attachable to the light source module, characterized in that
the light source module includes a light source, and a light source light emitting end which emits, to an outside, light source light emitted from the light source,
the irradiation module includes an optical connection terminal into which the light source light emitted from the light source module is applied, a light guide member which guides the light source light, and a light emitting member which emits the light source light,
the light source system includes light guide routes, each light guide route being an optical path which includes the light source, the light source light emitting end, the optical connection terminal, the light guide member, and the light emitting member, and allowing the light source light emitted from the light source module to pass therethrough, and
when a first light guide route is defined as a route comprising a first light source, a first light source light emitting end, a first optical connection terminal, a first light guide member, and a first light emitting member, and when a second light guide route is defined as a route comprising a second light source, a second light source light emitting end, a second optical connection terminal, a second light guide member, and a second light emitting member, the first light source light emitting end and the first optical connection terminal are constituted based on desirable first optical specifications, and the second light source light emitting end and the second optical connection terminal are constituted based on desirable second optical specifications, so that the first or second light guide route is constituted in the light source system.

According to the present invention, it is possible to construct light guide routes so that illuminating light for each purpose is emitted. In other words, light source modules connectable to irradiation modules each having a light guide member to appropriately guide light source light to be emitted are appropriately combined with the irradiation modules on which there can be mounted the light guide members to guide the light source light emitted from the light source modules, thereby emitting illuminating light for each purpose. Thus, it is possible to provide a light source system which enables the emission of the illuminating light for each of various purposes without having to construct an individual exclusive system.

### Brief Description of Drawings

FIG. 1 is a view showing a constitution of a light source system having light guide routes according to a first embodiment of the present invention;
FIG. 2 is a view showing a constitution of a modification of a second light source module;
FIG. 3(A) is a view showing electric connection terminals of a first light source module and a connecting portion, and FIG. 3(B) is a view showing electric connection terminals of the second light source module and a connecting portion;
FIG. 4 is a view showing a constitution of a light source system having light guide routes according to a second embodiment of the present invention including a fourth light source module;
FIG. 5A is a view showing a constitution of a fourth irradiation module in a light source system having light guide routes according to a third embodiment of the present invention;
FIG. 5B is a view showing cross sections of a first light guide member and a second light guide member constituted along the same axis in the fourth irradiation module;
FIG. 6 is a view showing a constitution of a modification of the fourth irradiation module;
FIG. 7 is a view showing constitutions of a fifth light source module and a fifth irradiation module in a light source system having light guide routes according to a fourth embodiment of the present invention; and
FIG. 8 is a view showing a constitution of a light source system having light guide routes according to a fifth embodiment of the present invention including a sixth irradiation module.

### Description of Embodiments

### [First Embodiment]

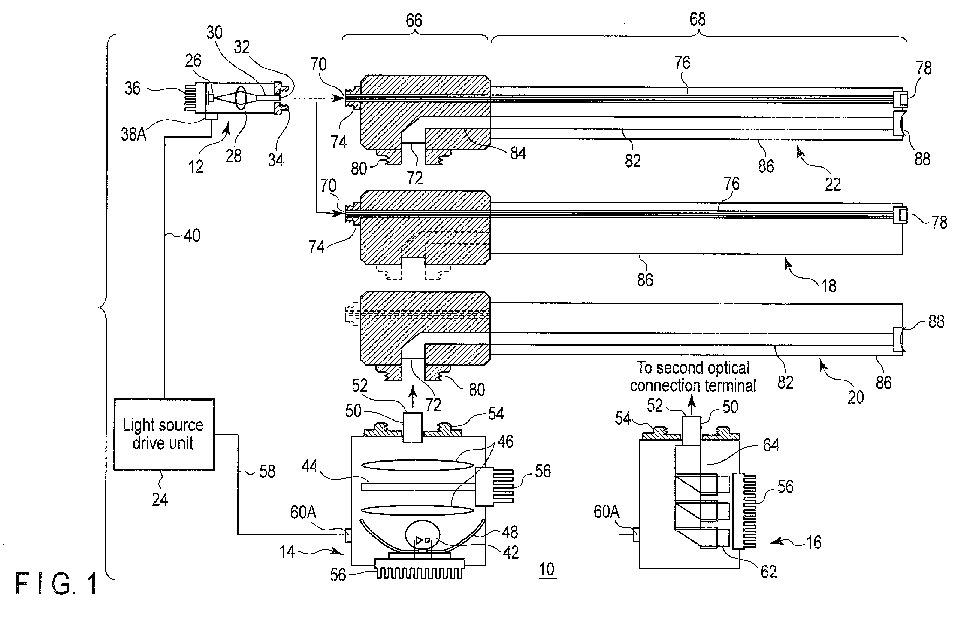

As shown in FIG. 1, a light source system 10 having light guide routes according to a first embodiment of the present invention is constituted by three light source modules (a first light source module 12, a second light source module 14, and a third light source module 16), and three irradiation modules (a first irradiation module 18, a second irradiation module 20, and a third irradiation module 22). That is, the separate light source modules 12, 14, and 16 and the separate irradiation modules 18, 20, and 22 are appropriately combined, so that it is possible to construct a light source device capable of emitting illuminating light for each purpose.

Next, respective elements constituting the present light source system 10 will be described in order.

First, the light source modules 12, 14, and 16 are described.

Each of the first to third light source modules 12, 14, and 16 has a light source which emits light source light, a light source light emitting end which emits the light source light, and a fixing portion A which attaches the light source light emitting end to a later-mentioned connection unit of the irradiation module. Furthermore, the light source module has an electric terminal which is electrically connected to a light source drive unit 24 to drive each light source, and receives, from the light source drive unit 24, a power and a control signal to allow the light source to emit the light.

Hereinafter, the separate light source modules 12, 14, and 16 will be described.

First, the first light source module 12 is described.

On the first light source module 12, a blue semiconductor laser light source 26 which emits blue laser light is mounted as a first light source. The blue laser light emitted from the blue semiconductor laser light source 26 is condensed via a lens 28, and a large part of the light enters a laser light guiding optical fiber 30. An emitting end of the laser light guiding optical fiber 30 is a first light source light emitting end 32. The first light source light emitting end 32 is constituted to be optically connectable to a later-mentioned first optical connection terminal of the connection unit (described later) of the irradiation module 18 or 22. The first light source module 12 and the connection unit have a mechanically holdable connection mechanism via no movable portion. That is, a first fixing portion A 34 disposed in the vicinity of the first light source light emitting end 32 of the first light source module 12 is constituted to engage with a later-mentioned first fixing portion B disposed in the vicinity of the first optical connection terminal of the connection unit, and the first fixing portions A and B constitute the mechanically holdable connection mechanism which connects the first light source module 12 and the connection unit via no movable portion.

Furthermore, the first light source module 12 has a heat radiation mechanism 36 in which a Peltier element and a heat radiation fin are combined to radiate heat generated from the blue semiconductor laser light source 26 as the first light source. In the drawing, for simplicity, only the heat radiation fins are depicted, and the Peltier element and a control system of the element are omitted.

Furthermore, the first light source module has an electric terminal 38A to be electrically connected to the light source drive unit 24 via a connection cable 40.

Next, the second light source module 14 will be described.

On the second light source module 14, a Xenon lamp 42, which is a type of electric discharge lamp, is mounted as a second light source. In the lamp light discharged from the Xe lamp 42, a forward discharged component is condensed via a filter 44 and lenses 46 and further, a rearward discharged component is condensed via a mirror 48, the filter 44 and the lenses 46, and part of these components enters a light guide rod 50. In the two lenses 46, the lens on the side of the Xe lamp 42 has a function of forming the lamp light into substantially parallel light, so that the lamp light formed into the substantially parallel light is applied onto the filter 44. The filter 44 is a band pass filter or a combination of a low pass filter and a high pass filter, which has a function of removing unnecessary ultraviolet and infrared rays from the lamp light discharged from the Xe lamp 42. In the two lenses 46, the lens disposed on the side of the light guide rod 50 has a function of condensing the lamp light from which unnecessary components are removed at an entrance end of the light guide rod 50. Furthermore, the mirror 48 is a concave surface mirror having a function of reflecting the rearward discharged lamp light toward the lenses 46 to condense the light on the lenses 46. An emitting end of the light guide rod 50 is a second light source light emitting end 52. The second light source light emitting end 52 is constituted to be optically connectable to a later-mentioned second optical connection terminal of the connection unit (described later) of the irradiation module 20 or 22. The second light source module 14 and the connection unit have a mechanically holdable connection mechanism via no movable portion. That is, a second fixing portion A 54 mounted on the second light source module 14 engages with a later-mentioned second fixing portion B mounted on the connection unit, to constitute the connection mechanism mechanically holding both of the second light source module 14 and connection mechanism via no movable portion. Furthermore, on the second light source module 14, a heat radiation mechanism is mounted in which a cooling fan and heat radiation fins 56 are combined to radiate heat generated from the Xe lamp 42, and heat of a member irradiated with the lamp light to absorb a part of the lamp light and raise its temperature. It is to be noted that in the drawing, for simplicity, only the heat radiation fins 56 are depicted, and the cooling fan, a related control system and the like are omitted.

Furthermore, the second light source module has an electric terminal 60A to be electrically connected to the light source drive unit 24 via a connection cable 58.

Next, the third light source module 16 will be described.

In the present embodiment, on the third light source module 16, LEDs 62 are mounted as a third light source in place of the Xe lamp 42 which is the second light source mounted on the second light source module 14. The third light source module 16 has a basic constitution similar to the second light source module 14, and is different in that the Xe lamp 42 is replaced with the LEDs 62 and in a constitution of an optical system for allowing the light source light to enter a light guide rod 50. The third light source module 16 is constituted so that the LEDs 62 of three colors of RGB are mounted on the same substrate, and LED light generated from the respective LEDs 62 is condensed in one optical path by a multiplexing optical system 64 to irradiate the light guide rod 50. The multiplexing optical system 64 is constituted by using dichroic mirrors or the like. As the substrate on which the LEDs 62 are mounted, an aluminum substrate, an aluminum nitride substrate or the like having a high thermal conductivity is used, and heat radiation fins 56 are disposed on a back surface of the substrate. It is to be noted that even in the case of the LEDs 62, heat of the LEDs 62 can more efficiently be radiated by using a cooling fan, and hence the cooling fan may be mounted. Furthermore, also in the case of the LEDs 62, a light condensing lens or a filter to remove unnecessary LED light may be used in accordance with a purpose.

Furthermore, in the same manner as in the above second light source module 14, the third light source module has an electric terminal 60A to be electrically connected to the light source drive unit 24 via the connection cable 58.

It is to be noted that the third light source module 16 has a basic functional constitution similar to the second light source module 14, and hence in the subsequent description, an example where the second light source module 14 is used will be described as a representative, and description of a constitution or an operation of the third light source module 16 is omitted.

Next, the light source drive unit 24 will be described.

The light source drive unit 24 is a unit which supplies power to the light source modules 12 and 14 (16) having such constitutions as described above, and controls emission states of the light sources to be mounted on the light source modules 12 and 14 (16). The light source system 10 in the present embodiment has the one light source drive unit 24 which is electrically connected to both of the first light source module 12 and the second light source module 14 via the connection cables 40 and 58 and the electric terminals 38A and 60A.

The light source drive unit 24 has a function of discriminating types of the connected light source modules 12 and 14 (16). That is, on the connection cables 40 and 58 of the light source drive unit 24 to the light source modules 12 and 14 (16), a discrimination signal wiring line to discriminate the types of the connected light source modules 12 and 14 (16) is mounted, in addition to a power wiring line to supply the power and a control signal wiring line to control drive states of the light sources. The light source drive unit 24 discriminates the types of the connected light source modules 12 and 14 (16) on the basis of a discrimination signal received via the discrimination signal wiring line. For example, when the connected light source module is the first light source module 12 on which the laser light source is mounted, the module is detected via the discrimination signal wiring line, and the power and control signal corresponding to the laser light source are supplied from the power wiring line and the control signal wiring line, respectively.

An example of a discrimination method of the connected light source module 12 or 14 (16) is a method in which the type or drive information of the light source is beforehand stored in an unshown storage memory in the light source module 12 or 14 (16), and read from the light source drive unit 24. Furthermore, as another method, there is contrived a method in which a terminal portion of the electric terminal 38A or 60A to be connected to the connection cable 40 or 58 is provided with a characteristic shape such as an uneven shape peculiar to the light source module, and the shape is detected by an unshown sensor mounted on the connection cable 40 or 58, to discriminate the type of the light source module 12 or 14 (16) on the basis of the detected shape. Furthermore, there is contrived a method or the like in which a unique electric signal pattern is output from the light source module 12 or 14 (16) through the discrimination signal wiring line, and the type of the connected light source module 12 or 14 (16) is discriminated from the electric signal pattern.

At this time, the control of the light source module 12 or 14 (16) is preferably appropriately regulated in accordance with not only the type of the light source module 12 or 14 (16) or a type of the irradiation module 18, 20, or 22 connected to the light source module 12 or 14 (16). Thus, it is necessary to transmit, to the light source drive unit 24, information of the type of the irradiation module 18, 20, or 22 connected to the light source module 12 or 14 (16). This information of the irradiation module 18, 20, or 22 may be transmitted via the light source module 12 or 14 (16) and the connection cable 40 or 58, or by directly connecting the light source drive unit 24 to the connection unit (described later).

It is to be noted that in the present embodiment, there has been described an example where the two connection cables 40 and 58 are connected from the light source drive unit 24 toward the two light source modules, but the present invention is not limited to this example. For example, a method is also possible in which the second light source module 14 is connected to the light source drive unit 24 via the connection cable 58, to control the first light source module 12 via the connection cable 58, the second light source module 14, and the connection unit connected to the second light source module 14. In this case, each of connecting portions of the first light source module 12 and the connection unit is provided with an electric connection terminal which is a control signal transmission terminal to transmit the control signal or a power supply terminal to supply the power, in addition to the first optical connection terminal (described later). At this time, this connection is preferably utilized for the discrimination signal of the type of the connected light source module.

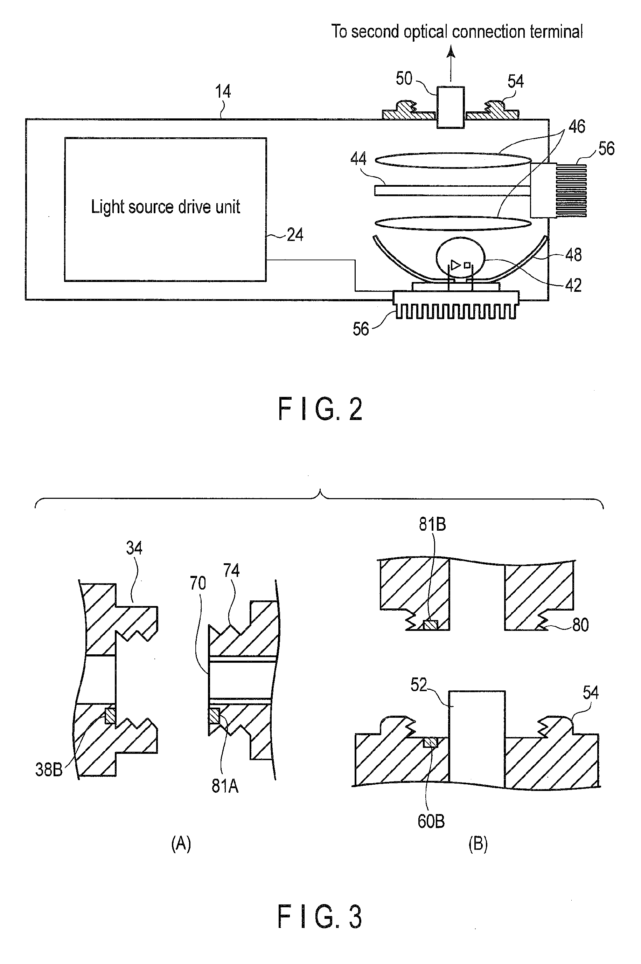

Furthermore, in the present embodiment, there has been described an example where the light source drive unit 24 is a unit separated from the light source modules 12 and 14 (16), but the present invention is not limited to this example. FIG. 2 shows a modification of the second light source module 14. In a constitution shown in FIG. 2, the light source drive unit 24 is mounted in the second light source module 14. The second light source module 14 has a large size and uses more power compared with the first light source module 12, and hence the constitution is not noticeably affected even when the light source drive unit 24 is mounted. In this case, the connection cable 40 to control the first light source module 12 is not used, but the first light source module is preferably controlled via the connection units of the irradiation modules 18, 20, and 22.

It is to be noted that the light source drive unit 24 can also be mounted on the first light source module 12. In this case, a difference between a size of the first light source module 12 + the light source drive unit 24 and a size of the second light source module 14 is small, and hence there is a merit that management of storing or the like is easily executed, or the like.

Next, the irradiation modules 18, 20, and 22 will be described.

Each of the first to third irradiation modules 18, 20, and 22 has a connection unit 66 and a light guide unit 68. In an end portion of the light guide unit 68, a light emitting member is disposed, and the light source light is formed into desirable illuminating light via this member, to irradiate an illumination object.

The present embodiment illustrates an example of three types of irradiation modules, 18, 20, and 22, in which a combination of types of the light guide units 68 to be mounted differ, i.e., a combination of types of emittable light differ.

Distinction of the three irradiation modules 18, 20, and 22 of the present embodiment will be described later, and elements of the irradiation modules 18, 20, and 22 are first described.

First, the connection unit 66 is described.

The connection unit 66 is a connecting portion optically and mechanically connected to the light source module 12, 14 or 16, to transmit, to the light guide unit 68, the light source light emitted from the light source module 12, 14 or 16. In the present embodiment, the connection unit 66 is a substantially rectangular parallelepiped body, and on two different flat surfaces, two optical connection terminals of a first optical connection terminal 70 and a second optical connection terminal 72 can be mounted.

The two optical connection terminals 70 and 72 disposed in the connection unit 66 are common in that both of the terminals have a function of transmitting, to the light guide unit 68, the light source light emitted from the light source. On the other hand, the two optical connection terminals 70 and 72 differ, as follows. That is, the first optical connection terminal 70 can mechanically be connected to the first light source module 12, but cannot mechanically be connected to the second light source module 14. The second optical connection terminal 72 can mechanically be connected to the second light source module 14, but cannot mechanically be connected to the first light source module 12.

In the present embodiment, the first optical connection terminal 70 is connected to the first light source module 12 on which the blue semiconductor laser light source 26 as the first light source is mounted. Therefore, the first optical connection terminal 70 is constituted based on desirable first optical specifications corresponding to the first light source light emitting end 32 of the first light source module 12. That is, the first light source light emitting end 32 and the first optical connection terminal 70 are constituted based on the desirable first optical specifications. Specifically, the first optical connection terminal 70 is constituted so that an end portion of a single optical fiber suitable for guiding the laser light is held by a ferrule. Further, in the vicinity of the first optical connection terminal 70, a first fixing portion B 74 is disposed, and constituted to engage with the first fixing portion A 34 disposed in the first light source module 12 to fix the portions to each other. That is, the first light source module 12 is mechanically connected to the connection unit 66 by a common connection mechanism. Preferable examples of the first optical connection terminal 70, the fixing portion A 34, and the fixing portion B 74 are FC connectors.

It is to be noted that without connecting the first light source module 12 to the light source drive unit 24 as described above, the light source drive unit 24 can be constituted to control the first light source module 12 via the connection unit 66, or via the second light source module 14 and the connection unit 66. In this case, as shown in FIG. 3(A), an electric connection terminal 81A and the first optical connection terminal 70 of the connection unit 66 are adjacently disposed on the same plane of the connection unit 66, and the above common connection mechanism mechanically connects the first light source module 12 to the connection unit 66, in a state where the first light source light emitting end 32 of the first light source module 12 is optically connected to the first optical connection terminal 70 and an electric connection terminal 38B of the first light source module 12 is electrically connected to the electric connection terminal 81A of the connection unit 66.

Furthermore, the first light source light emitting end 32 and/or the first optical connection terminal 70 may have an optical connection detecting mechanism to detect whether or not the connection unit 66 and/or the corresponding first light source module 12 is optically connected. In consequence, the light source drive unit 24 can be prevented from driving the first light source module 12 which is not connected to the irradiation module.

The single optical fiber is one optical fiber constituted by coating a core of a high refractive index with a clad of a lower refractive index. To guide the semiconductor laser light, an optical fiber having a core diameter of several µm to about 200 µm is suitable. For an illumination use application, a multimode optical fiber is suitable.

The end portion of the single optical fiber is mechanically held by the connection mechanism to face the laser light guiding optical fiber 30 of the first light source module 12 so that central axes substantially match each other. The single optical fiber passes through the inside of the connection unit 66, and extends to the light guide unit 68. That is, the single optical fiber, which is a first light guide member 76, substantially linearly extends in the connection unit 66 from the first optical connection terminal 70 to a first light emitting member 78.

On the other hand, the second optical connection terminal 72 is connected to the second light source module 14, which is a lamp light source. Therefore, the second optical connection terminal 72 is constituted based on desirable second optical specifications corresponding to the second light source light emitting end 52 of the second light source module 14. That is, the second light source light emitting end 52 and the second optical connection terminal 72 are constituted based on the desirable second optical specifications. Specifically, the second optical connection terminal 72 needs to receive and guide the light from a light source such as the Xe lamp 42 or the LEDs 62 whose light emission region is comparatively large, and hence the terminal has a comparatively large light entrance region as compared with the first optical connection terminal 70. In the vicinity of the second optical connection terminal 72, a second fixing portion B 80 is disposed, and constituted to engage with the second fixing portion A 54 disposed in the second or third light source module 14 or 16 to fix the portions to each other. That is, the second or third light source module 14 or 16 is mechanically connected to the connection unit 66 by a common connection mechanism.

It is to be noted that without connecting the second or third light source module 14 or 16 to the light source drive unit 24, the light source drive unit 24 can be constituted to control the second or third light source module 14 or 16 via the connection unit 66, or via the first light source module 12 and the connection unit 66. In this case, as shown in FIG. 3(B), an electric connection terminal 81B and the second optical connection terminal 72 of the connection unit 66 are adjacently disposed on the same plane of the connection unit 66, and the above common connection mechanism mechanically connects the second or third light source module 14 or 16 to the connection unit 66, in a state where the second light source light emitting end 52 of the second or third light source module 14 or 16 is optically connected to the second optical connection terminal 72 and an electric connection terminal 60B of the second or third light source module 14 or 16 is electrically connected to the electric connection terminal 81B of the connection unit 66.

Furthermore, the second light source light emitting end 52 and/or the second optical connection terminal 72 may have an optical connection detecting mechanism to detect whether or not the connection unit 86 and/or the corresponding second or third light source module 14 or 16 is optically connected. In consequence, the light source drive unit 24 can be prevented from driving the second or third light source module 14 or 16 which is not connected to the irradiation module.

Furthermore, to simultaneously enable the connection of both of the first light source module 12 and the second light source module 14, in the present embodiment, the first and second optical connection terminals 70 and 72 are disposed on two mutually perpendicular surfaces of the connection unit 66 having a substantially rectangular parallelepiped shape. As described later, in the light guide unit 68 of the third irradiation module 22, the first light guide member 76 and a second light guide member 82 are disposed in parallel, and hence in the connection unit 66, it is necessary to bend an optical path of one of the members substantially at right angles. Thus, in the present embodiment, as shown in FIG. 1, a bent light guide member 84 is mounted on the second optical connection terminal 72, and bends while guiding the light source light emitted from the second light source module 14, to guide the light to a light entrance end of the second light guide member 82. As the bent light guide member 84, it is possible to use an optical member or the like which is a rod of glass, plastic or the like and guides the light to the second light guide member 82 by utilizing total reflection.

It is to be noted that the bent light guide member 84 can be disposed at the first optical connection terminal 70, but when the member is disposed on the side of the second optical connection terminal 72 having a larger dimension, manufacturing is easier, costs are reduced, and a performance is increased.

Next, the light guide unit 68 will be described.

In the light guide unit 68, the light source light emitted from the light source is guided to the light emitting member. To emit the illuminating light at a desirable position or in a desirable direction, the light guide members 76 and 82 in the present embodiment are constituted to be freely curvable.

On the light guide unit 68, two light guide members, the first light guide member 76 and the second light guide member 82, can be mounted in parallel, and the unit is covered with a light guide unit cover 86. One end of the light guide unit 68 is connected to the connection unit 66, and the other end thereof extends to the light emitting member. In the light guide member mountable on the light guide unit 68, the single optical fiber which is the first light guide member 76 passes through the inside of the connection unit 66 from the first optical connection terminal 70, to extend in the light guide unit 68 to the first light emitting member 78. On the other hand, the second light guide member 82 extends in the light guide unit 68 from a position where the bent light guide member 84 disposed in the connection unit 66 faces the second light source light emitting end 52, to extend to a second light emitting member 88. In the second light guide member 82, a bundle fiber in which several hundred to several thousand bare optical fibers are bundled is used to guide the lamp light or LED light whose emission region is comparatively large. A light entrance end of the bundle fiber is made to abut on and is fixed to an emission surface of the bent light guide member 84.

The light guide unit 68 is constituted so that one or both of the single optical fiber as the first light guide member 76 and the bundle fiber as the second light guide member 82 can be mounted. That is, plural constitutions are enabled by a constitution of the light guide member to be mounted or the later-mentioned light emitting member.

It is to be noted that in the present embodiment, there has been described an example where the single optical fiber is used as the first light guide member 76 which is combined with the blue semiconductor laser light source 26 as the first light source to guide the blue laser light, and the bundle fiber in which the bare optical fibers are bundled is used as the second light guide member 82 which is combined with a light source such as the Xe lamp 42 or the LEDs 62 to guide the lamp light or the LED light, but the present invention is not limited to this example. For example, on the substrate or a film, an optical waveguide path obtained by patterning or laminating members having different refractive indexes can be used. In the optical waveguide path, light guide paths can be separated or combined, or various complicated optical paths can be patterned. Furthermore, as another example of the light guide path, a single optical fiber having a core diameter of several hundred microns or more can be used. When the core diameter is large, the light from the Xe lamp 42 or the LEDs 62 whose emission region is large can efficiently be guided. However, the single optical fiber having a large core diameter is not easily bent, and hence the fiber is not suitable when flexibility is required for the light guide unit 68.

Next, the light emitting members 78 and 88 will be described.

The light emitting member 78 or 88 is an optical function member having a function of converting the light source light emitted from the light source into desirable illuminating light. For example, the light emitting member has a function of controlling a luminous intensity distribution angle of the light source light emitted from the light guide member 76 or 82, or controlling a wavelength, a spectrum shape or an intensity of light.

The light emitting member 78 or 88 is disposed at an emission side end of the light guide unit 68.

In the present embodiment, as the first light emitting member 78, there is mounted a spectrum conversion member which absorbs a part of the blue laser light emitted from the blue semiconductor laser light source 26 as the first light source to emit converted yellow fluorescence, and scatters and emits the remaining blue laser light. As this spectrum conversion member, for example, a wavelength conversion member can be used in which a fluorescent powder of YAG:Ce or the like is dispersed in a resin or glass. By use of the wavelength conversion member, the peak wavelength, spectrum shape, or radiation angle of the blue laser light can be converted.

Furthermore, as the second light emitting member 88, there is mounted a radiation angle conversion member which is a type of luminous intensity distribution conversion member to enlarge an emission angle of the lamp light from the Xe lamp 42 as the second light source. In the radiation angle conversion member, for example, a concave lens is used. By the use of the radiation angle conversion member, the peak wavelength or spectrum shape of the lamp light from the Xe lamp 42 hardly changes, and the radiation angle can solely be converted.

It is to be noted that in the present embodiment, there has been described an example of the light guide unit 68 on which the above-mentioned spectrum conversion member and the above-mentioned luminous intensity distribution conversion member are mounted as the light emitting members 78 and 88, but the present invention is not limited to this example. For example, such various optical members as described in the following can be used as the light emitting members 78 and 88. Furthermore, these members can be combined and used.
(1) As the luminous intensity distribution conversion member, it is possible to use a radiation angle conversion member such as a convex lens or a combination of the convex lens and a concave lens, in addition to the concave lens, which converts the radiation angle, or a luminous intensity distribution conversion member such as a hologram lens or a diffraction lattice which changes the radiation angle or converts a direction of the light to be radiated, i.e., an orientation.
(2) As the luminous intensity distribution conversion member, it is possible to use a member in which particles of alumina or the like having a high refractive index and a high reflectance are dispersed in a resin or glass, a member in which transparent members having different refractive indexes are mixed, a scattering plate such as ground glass, a diffusion plate in which micro uneven portions are disposed in the surface, or the like.
(3) As the spectrum conversion member, in addition to a fluorescent body, it is possible to use an optical semiconductor, a member which generates secondary higher harmonic waves (SHG), an electroluminescence material, or the like.
(4) As an optical transmission modulation member which transmits a part of the light source light and blocks a part thereof, it is possible to use various optical filters, or a member such as a dyestuff or an optical resonator (etalon) having wavelength selecting properties.
(5) As the optical transmission modulation member which transmits a part of the light source light and blocks a part thereof, it is possible to use an optical switch, or a member such as an electrochromic member or a liquid crystal device having space selecting properties.

For example, (2) is suitable for safety of the laser light source or removal of speckles. Furthermore, when the radiation angle of the lamp light or the LED light is regulated, (1) or (2) can be used.

Next, the three irradiation modules 18, 20, and 22 will be described, respectively.

The light source system according to the present embodiment has the three irradiation modules 18, 20, and 22 as shown in FIG. 1.

First, the third irradiation module 22 is described. In the third irradiation module 22, both of the first optical connection terminal 70 and the second optical connection terminal 72 disposed in the connection unit 66 are valid, so that the first optical connection terminal 70 is connectable to the first light source module 12, and the second optical connection terminal 72 is connectable to the second light source module 14. From the first optical connection terminal 70, the single optical fiber extends as the first light guide member 76, and at the light emitting end of the single optical fiber, the fluorescent body is mounted as the wavelength conversion member which is the first light emitting member 78. Furthermore, the bent light guide member 84 and the bundle fiber as the second light guide member 82 are connected in order from the second optical connection terminal 72, and at the light emitting end of the bundle fiber, the concave lens which is a radiation angle control member as the second light emitting member 88 is mounted. The first and second light guide members 76 and 82 mounted on the light guide unit 68 of the third irradiation module 22 are covered with the common light guide unit cover 86.

On the light source device constructed by attaching both of the first light source module 12 and the second light source module 14 to the third irradiation module 22, light guide routes are mounted. In other words, on such a light source device, a first light guide route is mounted to be linked to the blue semiconductor laser light source 26 which is the first light source, the first light source light emitting end 32, the first optical connection terminal 70, the first light guide member 76, and the first light emitting member 78. Further, on such a light source device, a second light guide route is mounted to be linked to the Xe lamp 42 which is the second light source, the second light source light emitting end 52, the second optical connection terminal 72, the second light guide member 82, and the second light emitting member 88.

Furthermore, in the first irradiation module 18, only the first optical connection terminal 70 is valid in the first optical connection terminal 70 and the second optical connection terminal 72 disposed in the connection unit 66, and the bent light guide member 84 to guide the light is not mounted on the second optical connection terminal 72. In the subsequent stage to the valid first optical connection terminal 70, the first light guide member 76 and the fluorescent body which is the first light emitting member 78 are mounted in the same manner as in the third irradiation module 22. That is, on a combination of the first irradiation module 18 and the first light source module 12, the above-mentioned first light guide route is mounted, but the second light guide route is not mounted.

Additionally, in the second irradiation module 20, only the second optical connection terminal 72 is valid in the first optical connection terminal 70 and the second optical connection terminal 72 disposed in the connection unit 66, and the first optical connection terminal 70 is not connected to the first light guide member 76. In the subsequent stage to the valid second optical connection terminal 72, the bent light guide member 84, the second light guide member 82, and the concave lens which is the second light emitting member 88 are mounted in the same manner as in the third irradiation module 22. That is, on a combination of the second irradiation module 20 and the second light source module 14, the above-mentioned first light guide route is not mounted, but the second light guide route is mounted.

It is to be noted that in the first and second irradiation modules 18 and 20 shown in FIG. 1, the optical connection terminals which are not valid are shown by broken lines, respectively, and there may or may not be disposed a fixing structure to mechanically fix the light source module 12, 14 or 16 to this optical connection terminal that is not valid. That is, for example, when a die forming technique is used in preparation of a case body of the connection unit 66, separate preparation of a die excluding a portion of the optical connection terminal 70 or 72 imposes a burden on costs and the number of steps. Therefore, when the first fixing portion B 74 or the second fixing portion B 80 is disposed in the vicinity of the optical connection terminal which is not valid, no problems occur. Furthermore, in a case where the connection unit 66 is to be miniaturized as much as possible in accordance with a user's use application, or in a case of a preparation method where the connection unit 66 is prepared as a member which is separate from the fixing portions B 74 and 80 to mechanically fix the light source module 12, 14 or 16, it is not necessary to dispose the fixing portion B 74 or 80 corresponding to the optical connection terminal which is not valid. However, in any of the cases, a countermeasure is preferably performed so that the light source module 12, 14 or 16 is not attached, by mistake, to the optical connection terminal which is not valid. For example, a lid is preferably disposed so that the optical connection terminal cannot be inserted. Otherwise, a projection or the like which prevents the light source module 12, 14 or 16 from engaging with the connection unit 66 is preferably disposed in one of the light source module 12, 14 or 16 and the connection unit 66. In addition to these countermeasures, display is preferably performed by color, an indicator or the like so that the optical connection terminal which is not valid can easily be identified.

Next, an operation of the light source system 10 of the present embodiment will be described.

First, there is described the operation when the first light source module 12 is connected to the third irradiation module 22 on which the first light guide member 76 and the second light guide member 82 are mounted.

The first light source module 12 is connected to the third irradiation module 22, whereby the first light guide route is constituted. The blue semiconductor laser light source 26 which is the first light source mounted on the first light source module 12 emits the blue laser light in response to the control signal from the light source drive unit 24 connected to the first light source module 12. The blue laser light emitted from the blue semiconductor laser light source 26 is applied onto the wavelength conversion member which is the first light emitting member 78 disposed at the light emitting end of the first light guide member 76, along the first light guide route, i.e., via the laser light guiding optical fiber 30, the first light source light emitting end 32, the first optical connection terminal 70, and the first light guide member 76. The wavelength conversion member absorbs a part of the blue laser light to convert the wavelength into that of the yellow fluorescence, and diffuses and transmits the remaining part. A light intensity ratio between the blue laser light and the yellow fluorescence is set so that the mixed light becomes white light, by regulating a thickness, concentration, shape or the like of the fluorescent body. In the semiconductor laser, as compared with a laser light source such as the Xe lamp 42 or the LEDs 62, extraordinary miniaturization and power saving are possible, and hence the light source device constructed by this combination is a small and highly efficient light source device. Therefore, the light source device is very effective for a use application in which the device is to be miniaturized or a use application in which battery drive or the like is required.

Next, there will be described an operation of a light source device in which the second light source module 14 is combined with the third irradiation module 22.

The second light source module 14 is connected to the third irradiation module 22, whereby the second light guide route is constituted. The Xe lamp 42, which is the second light source mounted on the second light source module 14, emits the lamp light in response to the control signal from the light source drive unit 24 connected to the second light source module 14. The lamp light emitted from the Xe lamp 42 is applied onto the concave lens as the radiation angle control member which is the second light emitting member 88 disposed at the light emitting end of the second light guide member 82, along the second light guide route, i.e., via the light guide rod 50, the second light source light emitting end 52, the second optical connection terminal 72, and the second light guide member 82. The concave lens spreads the lamp light at a desirable spread angle to emit the light toward an unshown illumination object.

The lamp light from the Xe lamp 42 has a spectrum comparatively close to sunlight, and is therefore effective when observation using light close to sunlight is required.

Next, there will be described an operation of a light source device in which the third light source module 16 and the third irradiation module 22 are combined.

The third light source module 16 is connected to the third irradiation module 22, whereby the second light guide route is constituted. The LEDs 62, which are the third light source mounted on the third light source module 16, emit the LED light in response to the control signal output from the light source drive unit 24 connected to the third light source module 16. The subsequent operation is similar to the operation in the case where the second light source module 14 is connected, and the illuminating light is emitted via the second light guide route.

In the LEDs 62, light of various emission colors can be utilized. Furthermore, when the multiplexing optical system 64 is used as in the present embodiment, it is possible to emit the light of various colors required for the illumination. Furthermore, as compared with the semiconductor laser, a size is large and power consumption is also large, but as compared with the lamp light source, the size is small and power saving is achieved.

Next, there will be described an operation in a case where the first light source module 12 is connected to the first irradiation module 18.

The first irradiation module 18 is a constitution where the members concerned with the second light guide route are removed from the third irradiation module 22. Therefore, a basic operation and an effect are similar to those in the case where the first light source module 12 is connected to the third irradiation module 22.

The first irradiation module 18 is used, which produces an effect that the device can be prepared more inexpensively and with less weight, commensurate with the reduction in members concerned with the second light guide route, in addition to the effect of the combination of the first light source module 12 and the third irradiation module 22. Furthermore, if necessary, the light guide unit 68 can be made to be thin.

Finally, there will be described an operation in the case where the second light source module 14 is connected to the second irradiation module 20.

The second irradiation module 20 is a constitution where the members concerned with the first light guide route are removed from the third irradiation module 22. Therefore, a basic operation and an effect are similar to those in the case where the second light source module 14 is combined with the third irradiation module 22.

The second irradiation module 20 is used, whereby the device can be prepared more inexpensively and with less weight, commensurate with the reduction in members concerned with the first light guide route, while various light sources such as the Xe lamp 42 and the LEDs 62 can be utilized. Furthermore, if necessary, a light guide unit 68 portion can be made to be thin.

As described above, in the light source system 10 according to the present embodiment, the light guide routes are set in accordance with the types of the light sources, and the light guide route suitable for the purpose can be selected, so that the combination of the light source device to be realized can freely be selected. At this time, the common light guide route can be used for the light sources which emit the light of similar characteristics, for example, the LEDs 62 and the Xe lamp 42, so that it is not necessary to unnecessarily increase the number of the light guide routes. That is, for the light sources in which emission points are comparatively large and emission characteristics are close to each other, for example, the Xe lamp 42 and the LEDs 62, optical specifications of the optical connection terminals are set in common, i.e., the second light source light emitting end 52 and the second optical connection terminal 72 are constituted based on the desirable second optical specifications. Consequently, the LEDs 62 and the Xe lamp 42 can use the common light guide route.

Moreover, when one set of the light source module and the irradiation module which conforms to the present light source system 10 is acquired, a light source module and an irradiation module which are usable in combination with this set can be additionally purchased, so that the system can be expanded, and the illuminating light suitable for the purpose can easily be obtained.

In consequence, it is possible to construct the light source system which can cope with various purposes without constructing an individual exclusive system. Furthermore, it is possible to obtain the illuminating light suitable for the purpose with good cost performance.

### [Second Embodiment]

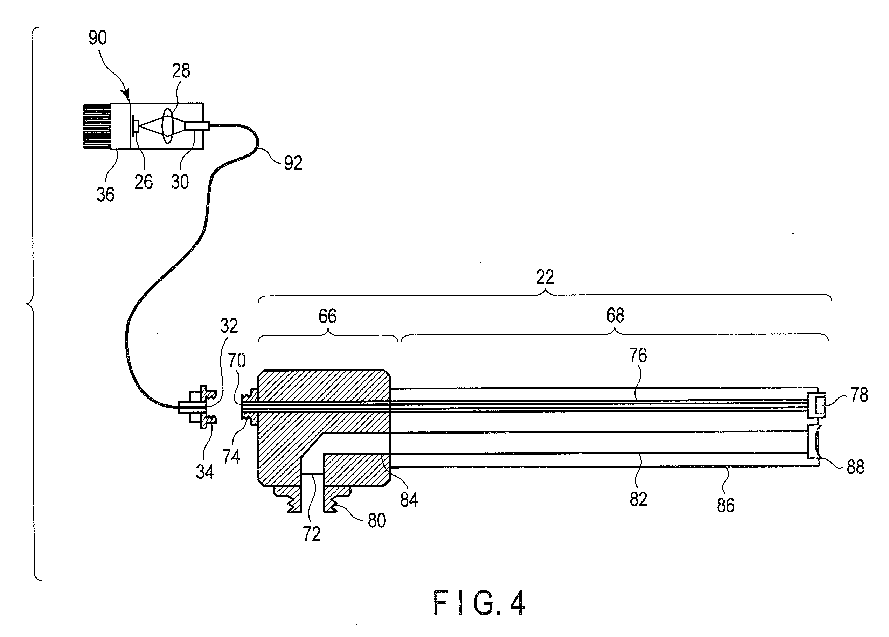

Next, a light source system having light guide routes according to a second embodiment of the present invention will be described with reference to FIG. 4.

It is to be noted that description of parts similar to those of the above first embodiment is omitted, and only different parts will be described.

A light source system 10 according to the present embodiment is different from the above first embodiment in the use of a fourth light source module 90 constituted by separating a first light source light emitting end 32 in a first light source module 12 from a blue semiconductor laser light source 26 which is a first light source, and connecting both to each other by an optical fiber.

That is, the first light source module 12 has been constituted so that the blue semiconductor laser light source 26 which is the first light source and the first light source light emitting end 32 are fixed to a common case body, and are connected to a connection unit 66 via no movable portion. On the contrary, in the fourth light source module 90, the blue semiconductor laser light source 26 which is the first light source is connected to the first light source light emitting end 32 via a bendable single optical fiber 92. That is, a main body of the fourth light source module 90 is connected to the first light source light emitting end 32 via a movable portion such as the optical fiber. In other words, the fourth light source module 90 is constituted so that a main body portion on which the light source is mounted is movably connected to the first light source light emitting end 32 to be connected to the connection unit 66, via the single optical fiber 92.

On the other hand, in the fourth light source module 90, almost all generated heat has to be radiated from the fourth light source module 90 to the outside, and hence as compared with the first light source module 12, a radiation capability of a heat radiation mechanism 36 is preferably enhanced.

The first light source light emitting end 32 is provided with a fixing structure where there is disposed a first fixing portion A 34 which fixes the light emitting end optically connected to a first optical connection terminal 70, to be fixable by engaging with a first fixing portion B 74 disposed in the vicinity of the first optical connection terminal 70 of the connection unit 66. For example, the first light source light emitting end 32 and the first optical connection terminal 70 are FC connectors.

It is to be noted that in FIG. 4, for simplicity, only an example of a third irradiation module 22 is shown, but any irradiation module can be used as long as the module has a first light guide route. For example, a first irradiation module 18 can be utilized.

A basic operation of the light source system 10 according to the present second embodiment of the above-mentioned constitution is similar to that of the above first embodiment.

As described above, in the light source system 10 according to the present second embodiment, the irradiation module, for example, the first or third irradiation module 18 or 22 is connected to the fourth light source module 90 via the movable portion, and hence a degree of freedom in the arrangement of the light source modules is high. Furthermore, less members are fixed to the irradiation module, and hence as compared with the first embodiment, it is easy to handle the irradiation module.

It is to be noted that in the present embodiment, as to the light source module to be connected to the first light guide route, there has been described the example where the fourth light source module 90 is connected to the connection unit 66 via the movable portion, but the present invention is not limited to this example. A second or third light source module 14 or 16 which is the light source module to be connected to a second light guide route can be connected to the connection unit 66 via a movable portion. At this time, a light guide member for use as the movable portion is preferably a bundle fiber in which bare fibers are bundled.

### [Third Embodiment]

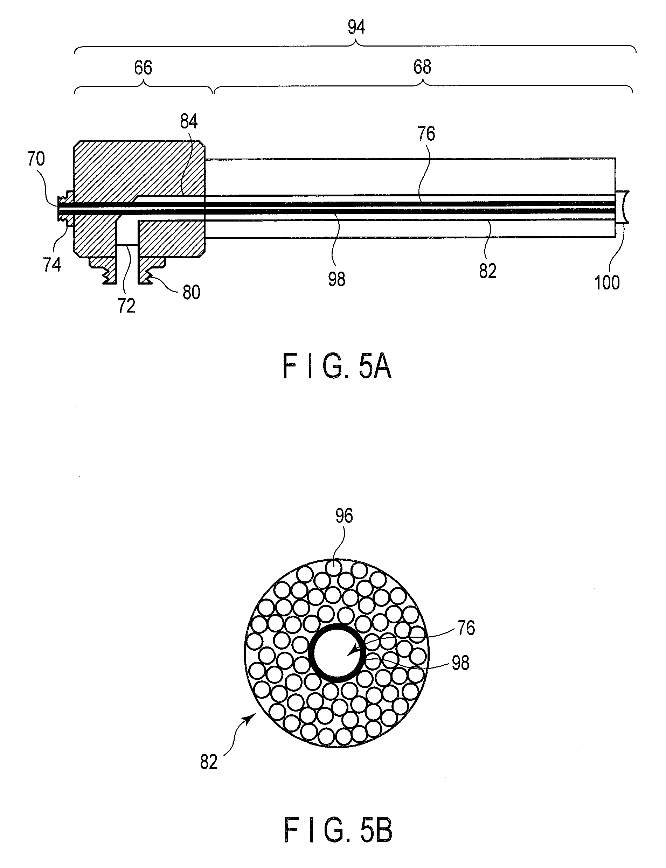

Next, a light source system having light guide routes according to a third embodiment of the present invention will be described with reference to FIG. 5A and FIG. 5B.

The present third embodiment is different from the above first embodiment in including a fourth irradiation module 94 on which a first light guide member 76 and a second light guide member 82 are coaxially mounted, and the embodiment is similar to the above first embodiment in the other parts. Therefore, description of the parts similar to those of the above first embodiment is omitted, and only different parts will be described.

As to the fourth irradiation module 94, in a light guide unit 68, the first light guide member 76 and the second light guide member 82 are coaxially constituted. That is, a periphery of the first light guide member 76 which is a single optical fiber is surrounded with the second light guide member 82 which is a bundle fiber constituted of bare optical fibers 96. A cross section of the coaxially constituted first light guide member 76 and second light guide member 82 is shown in FIG. 5B. It is to be noted that the single optical fiber which is the first light guide member 76 is covered with an optical fiber cover 98 so that blue laser light emitted from a first light source module 12 does not leak out to the bundle fiber which is the second light guide member 82.

The single optical fiber which is the first light guide member 76 passes through the inside of a bent light guide member 84 from a first optical connection terminal 70, and extends in the light guide unit 68 to reach a light emitting member 100.

The single optical fiber and the bundle fiber are disposed along the same axis, but the blue laser light emitted from the first light source module 12 travels only through the single optical fiber which is the first light guide member 76, and lamp light or LED light emitted from a second or third light source module 14 or 16 travels only through the bundle fiber which is the second light guide member 82. Therefore, the first light guide route is different from the second light guide route. In the present embodiment, a first light emitting member 78 which is an outlet of a first light guide route and a second light emitting member 88 which is an outlet of a second light guide route are constituted as the common light emitting member 100. That is, in a light source device constructed by combining the first light source module 12 and the second light source module 14 with the fourth irradiation module 94, the first light guide route is mounted to be linked to a blue semiconductor laser light source 26 as a first light source, a first light source light emitting end 32, the first optical connection terminal 70, the first light guide member 76, and the first light emitting member 78. Furthermore, the second light guide route is mounted to be linked to an Xe lamp 42 as a second light source, a second light source light emitting end 52, a second optical connection terminal 72, the second light guide member 82, and the second light emitting member 88. At this time, the first light emitting member 78 and the second light emitting member 88 constitute the light emitting member 100 which is a common concave lens.

A basic operation of a light source system 10 according to the present third embodiment of such a constitution as described above is similar to that of the above first embodiment.

As described above, in the present third embodiment, the first light guide member 76 and the second light guide member 82 are coaxially formed, and hence the first light emitting member 78 and the second light emitting member 88 are apparently at the same position. Furthermore, the first light emitting member 78 and the second light emitting member 88 form the light emitting member 100 which is the common concave lens. Therefore, the light emitted from the first light source module 12 and the light emitted from the second light source module 14 are apparently emitted from the same point of the fourth irradiation module 94. Therefore, in a case where the light source modules 12, 14, and 16 are switched and used, or the like, a way to form a shade, a luminous intensity distribution, or the like does not easily change, which produces an effect that observation can easily be carried out. Furthermore, when a lens, a filter or the like to regulate the luminous intensity distribution is added, these members can be constituted as a common member. That is, as the light emitting member 100 of the two light guide members 76 and 82, the common lens or filter can be disposed.

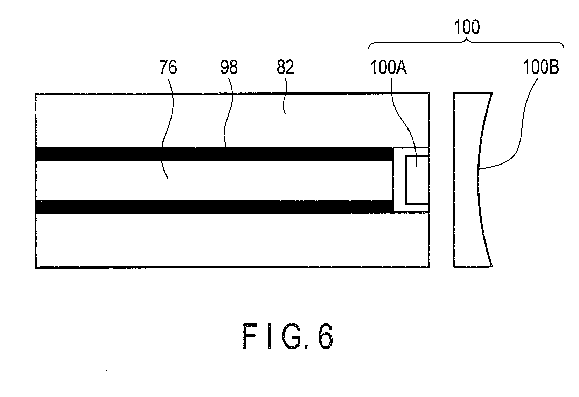

It is to be noted that in the present third embodiment, there has been described an example where the common concave lens is disposed as the light emitting member 100 for both of the first light guide member 76 and the second light guide member 82. As a modification of this embodiment, FIG. 6 shows an example of the light emitting member 100 in which a wavelength conversion member is mounted on a tip portion of the first light guide member 76.

In the example shown in FIG. 6, at a light emitting end of the first light guide member 76, a wavelength conversion member 100A is mounted, which is the same first light emitting member 78 as in the above first embodiment. Furthermore, at emitting ends of both of the first light guide member 76 and a second light guide member 82, a concave lens 100B which is a radiation angle control member is mounted. That is, the light emitting member 100 includes the wavelength conversion member 100A optically connected to the first light guide member 76, and the concave lens 100B optically separated from the second light guide member 82. Consequently, a function similar to that of a third irradiation module 22 can be constituted by disposing the first light guide member 76 and the second light guide member 82 along the same axis. In consequence, illuminating light similar to that of the above first embodiment can coaxially be emitted. It is to be noted that in the above first embodiment, the wavelength conversion member which is the first light guide member 76 is constituted to emit the illuminating light via no concave lens, but in the present modification, for example, the concave lens 100B is disposed. Therefore, to obtain a necessary radiation angle, a thickness, shape or the like of a fluorescent body to be mounted on the wavelength conversion member 100A appropriately needs to be regulated.

It is to be noted that also in the present third embodiment and the modification of the embodiment, needless to say, the fourth light source module 90 described in the above second embodiment may be used.

### [Fourth Embodiment]

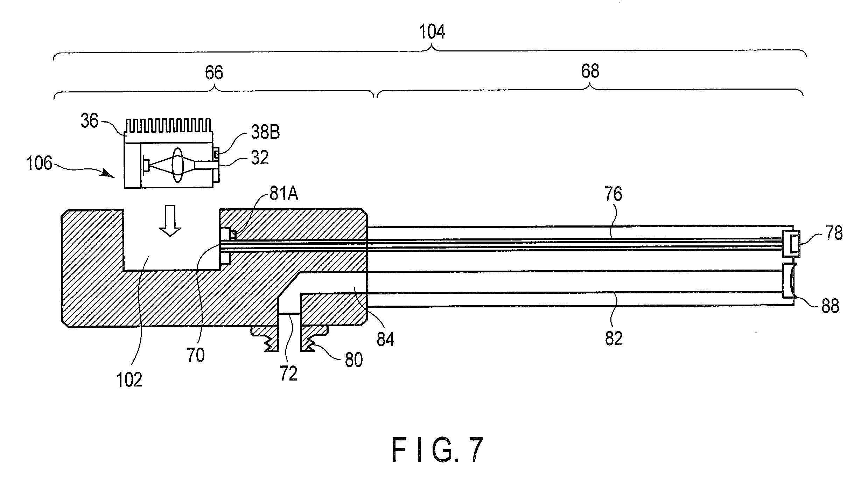

Next, a light source system having light guide routes according to a fourth embodiment of the present invention will be described with reference to FIG. 7.

The present fourth embodiment is different from the above first embodiment in the use of a fifth irradiation module 104 which is a configuration where a dented portion 102 is disposed in a connection unit 66 of a third irradiation module 22, and a fifth light source module 106 constituted in a configuration where a first light source module 12 can directly be mounted in the dented portion 102, and the other constitution is similar to the above first embodiment.

According to such a constitution, the fifth light source module 106 and the fifth irradiation module 104 can be handled as an integral member, which produces a merit that moving and storing are easily performed.

Here, a power of the fifth light source module 106 may be supplied via the dented portion 102 of the fifth irradiation module 104. For example, an electric connection terminal 81A is disposed on the same surface as in a first optical connection terminal 70 of the dented portion 102, a first light source light emitting end 32 is optically connected to the first optical connection terminal 70, and an electric connection terminal 38B of the fifth light source module 106 is electrically connected to the electric connection terminal 81A of the dented portion 102 of the connection unit 66. In this state, the fifth light source module 106 and the connection unit 66 can mechanically be connected by a common connection mechanism.

It is to be noted that in FIG. 7, for simplicity, an example of the fifth irradiation module 104 corresponding to the third irradiation module 22 is only shown, but any irradiation module can be used as long as the module has the first light guide route. For example, the dented portion 102 may be an inner surface in a connection unit 66 of a first irradiation module 18.

### [Fifth Embodiment]

In such a light source system having light guide routes as described in each of the above first to fourth embodiments, an image acquisition unit to acquire an image can be incorporated and used in each of the first to fifth irradiation modules 18, 20, 22, 94, and 104.

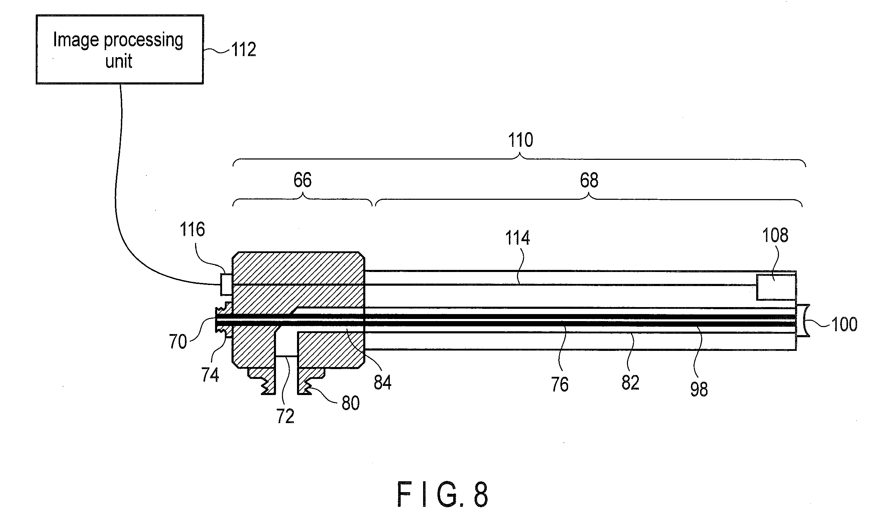

For example, FIG. 8 is a view showing a light source system 10 constituted of a sixth irradiation module 110 in which an image acquisition unit 108 is mounted on a fourth irradiation module 94 described in the above third embodiment, and an image processing unit 112 having a function of processing an image signal acquired by the image acquisition unit 108.

A light guide unit 68 of this sixth irradiation module 110 has a signal transmission member 114 in which the image acquisition unit 108 is disposed close to a light emitting member 100 and which transmits the image signal acquired by the image acquisition unit 108. A connection unit 66 has an image signal terminal 116 capable of transmitting, to the image processing unit 112, the image signal transmitted by the signal transmission member 114, and is constituted to be detachably attached to the image processing unit 112 by a portion of the image signal terminal 116.

The image signal acquired by the image acquisition unit 108 is transmitted to the image processing unit 112 via the signal transmission member 114 disposed in the light guide unit 68 and the image signal terminal 116 disposed in the connection unit 66. That is, the light source system having light guide routes according to the present fifth embodiment has an image signal transmission route which reaches the image processing unit 112 via the image acquisition unit 108, the signal transmission member 114, and the image signal terminal 116.

According to such a constitution, an image by illuminating light applied from a light source device constructed by the light source system 10 of the present fifth embodiment can stably and easily be acquired. That is, the image acquisition unit 108 and the light emitting member 100 are closely disposed and fixed to the light guide unit 68 which is a mutually common case body, so that an emitting position of the illuminating light does not shift from an image acquiring position of the image acquisition unit 108, and a light intensity, luminous intensity distribution or the like of the illuminating light on the image does not easily deviate. Furthermore, in the aspect of an operation, as compared with a case where the image acquisition unit 108 is mounted in a case body separate from the irradiation module, the operation is also easier, because they are integrally formed in the present fifth embodiment.

Furthermore, for obtaining the illuminating light suitable for image observation, an appropriate light source module and an appropriate irradiation module can be combined and utilized. As a result, various images can easily be obtained in accordance with purposes.

It is to be noted that the above description is made concerning a constitution corresponding to the fourth irradiation module 94 in the third embodiment as an example. However, needless to say, the image acquisition unit 108 can be disposed close to the light guide unit 68 of each of the first to third irradiation modules 18, 20, and 22 and the fifth irradiation module 104 according to the above first, second, and fourth embodiments, i.e., close to the first light emitting member 78 or the second light emitting member 88. Furthermore, it is possible to combine various light source modules which realize brightness, color or the like of the illuminating light suitable for an imaging purpose of endoscope observation or the like.

The present invention has been described above on the basis of the first to fifth embodiments, but each of the above-mentioned light source systems according to the five embodiments is merely one example, and it is possible to realize a light source system in which all of these systems are combined, or several constitutional elements can appropriately be selected. Furthermore, various changes are possible without departing from the gist of the present invention.

For example, in the above description, there has been described, as the example of the first light guide route, the example of the use of the blue semiconductor laser light source 26 which is the first light source, the single optical fiber which is the first light guide member 76, and the fluorescent body which is the first light emitting member 78, but the present invention is not limited to this example. As the first light source, semiconductor laser of blue purple or the like, i.e., color other than blue is used, and combined with a single optical fiber and a fluorescent body, whereby it is possible to realize a light source having a different color or different color rendering properties. Furthermore, for a purpose of illumination with a single color, it is possible to use, as the first light source, one of various laser light sources such as various semiconductor lasers, solid state lasers and gas lasers including other colors. Furthermore, it is possible to use, as the first light source, a white laser, a super continuum light source or the like.

## Claims

1. A light source system (10) constituted by combining:
a light source module (12; 14; 16; 90; 106), and
an irradiation module (18; 20; 22; 94; 104; 110) mechanically detachably attachable to the light source module, **characterized in that**
the light source module includes a light source, and a light source light emitting end which emits, to an outside, light source light emitted from the light source,
the irradiation module includes an optical connection terminal into which the light source light emitted from the light source module is applied, a light guide member which guides the light source light, and a light emitting member which emits the light source light,
the light source system includes light guide routes, each light guide route being an optical path which includes the light source, the light source light emitting end, the optical connection terminal, the light guide member, and the light emitting member, and allowing the light source light emitted from the light source module to pass therethrough, and
when a first light guide route is defined as a route comprising a first light source (26), a first light source light emitting end (32), a first optical connection terminal (70), a first light guide member (76), and a first light emitting member (78; 100), and when a second light guide route is defined as a route comprising a second light source (42; 62), a second light source light emitting end (52), a second optical connection terminal (72), a second light guide member (82), and a second light emitting member (88; 100), the first light source light emitting end and the first optical connection terminal are constituted based on desirable first optical specifications, and the second light source light emitting end and the second optical connection terminal are constituted based on desirable second optical specifications, so that the first or second light guide route is constituted in the light source system.

2. The light source system according to claim 1, **characterized in that**
the irradiation module is constituted by a connection unit (66) on which the optical connection terminal is mounted, and a light guide unit (68) on which the light guide member is mounted, and
at least one of the first optical connection terminal and the second optical connection terminal is mounted on the connection unit in accordance with the light guide route to be mounted.

3. The light source system according to claim 2, **characterized in that**
by number and types of light guide members to be mounted in accordance with corresponding light guide route, the irradiation module is one of:
a first irradiation module (18) on which only the first light guide member is mounted;
a second irradiation module (20) on which only the second light guide member is mounted; and
a third irradiation module (22; 94; 104; 110) on which both of the first light guide member and the second light guide member are mounted.

4. The light source system according to claim 2 or 3, **characterized in that**
in accordance with corresponding light guide route, the light source module is one of:
a first light source module (12; 90; 106) connectable only to the first optical connection terminal; and
a second light source module (14; 16) connectable only to the second optical connection terminal.

5. The light source system according to claim 4, **characterized in that**
the first optical specifications and the second optical specifications are different from each other, and
as compared with a valid light entrance region of the first optical connection terminal, a valid light entrance region of the second optical connection terminal is large.

6. The light source system according to claim 2, **characterized in that**
the first light guide member is a single optical fiber, and
the second light guide member is a bundle fiber constituted by bundling bare optical fibers (96).

7. The light source system according to claim 6, **characterized in that**
the connection unit has at least two surfaces of a first surface and a second surface,
the first optical connection terminal is mounted on the first surface, and
the second optical connection terminal is mounted on the second surface.

8. The light source system according to claim 7, **characterized in that**
a central axis in the vicinity of a light source light entrance end of the second light guide member is substantially perpendicular to an emitting direction of second light source light to enter the second optical connection terminal, and
a bent light guide member (84) is disposed on the second light guide route between the light source light entrance end of the second light guide member and the second optical connection terminal.

9. The light source system according to claim 8, **characterized in that** a central axis in the vicinity of a light source light entrance end of the first light guide member substantially matches an emitting direction of first light source light to enter the first optical connection terminal.

10. The light source system according to claim 9, **characterized in that**
the first light source is a laser or super luminescent light source, and
the second light source is a light source whose emission region is larger than that of the first light source.

11. The light source system according to claim 10, **characterized in that** the second light source is one of LEDs, a discharge type lamp, and a filament type lamp.

12. The light source system according to claim 6, **characterized in that** the second light source module is connected to the second optical connection terminal via no movable portion.

13. The light source system according to claim 12, **characterized in that** the first light source module is connected to the first optical connection terminal via no movable portion.

14. The light source system according to claim 13, **characterized in that**
the connection unit has a dented portion (102) in which the first light source module (106) is mountable, and
the first light source module is mounted in the dented portion.

15. The light source system according to claim 12, **characterized in that**
the first light source module (90) is connected to the connection unit via an optical fiber (92), and
the optical fiber is disposed on the first light guide route between the first light source light emitting end and the first optical connection terminal.

16. The light source system according to claim 12, **characterized in that**
the connection unit includes an electric connection terminal (81A), and
the first light source module includes an electric connection terminal (38B) electrically connectable to the electric connection terminal of the connection unit, and a power is supplied from a side of the connection unit via the electric connection terminals.

17. The light source system according to claim 16, **characterized in that**
the electric connection terminal of the connection unit and the first optical connection terminal are adjacently disposed on a same surface of the connection unit, and
in a state where the first light source light emitting end is optically connected to the first optical connection terminal and the electric connection terminal of the first light source module is electrically connected to the electric connection terminal of the connection unit, a common connection mechanism (34, 74) mechanically connects the first light source module to the connection unit.

18. The light source system according to claim 6, **characterized in that** on the light emitting member, there is mounted an optical function member which converts at least one of a peak wavelength, a spectrum shape, a luminous intensity distribution angle, and an intensity of light in optical properties of the light source light.

19. The light source system according to claim 18, **characterized in that** the optical function member (78) is a spectrum conversion member which receives the light source light and converts the light source light into a light of a different spectrum.

20. The light source system according to claim 18, **characterized in that** the optical function member (88) is a luminous intensity distribution conversion member which receives the light source light and converts the light source light into a light of a different luminous intensity distribution.

21. The light source system according to claim 18, **characterized in that** the optical function member is an optical transmission modulation member which receives the light source light, transmits a part of the light source light and blocks a part of the light source light.

22. The light source system according to claim 18, **characterized in that**
the first light guide member is surrounded with the second light guide member, and
the optical function member (100) includes a member (100A) optically connected to the first light guide member, and a member (100B) optically separated from the second light guide member.

23. The light source system according to claim 6, **characterized in that** each of the first optical connection terminal and the second optical connection terminal includes an optical connection detecting mechanism which detects whether or not the corresponding first light source module or the corresponding second light source module is optically connected.

24. The light source system according to claim 6, **characterized in that** the light source system includes a light source control unit (24) configured to control emission states of both of the first light source module and the second light source module.

25. The light source system according to claim 24, **characterized in that**
the light source control unit is mounted in a case body common to one of the first light source module and the second light source module, and
an emission state of other light source module is controllable via the connection unit.

26. The light source system according to claim 24, **characterized in that**
the light source control unit and the light source module are connected by a discrimination signal wiring line, and
the light source control unit discriminates a type of the light source module by a signal obtained by the discrimination signal wiring line, and executes control corresponding to the type.

27. The light source system according to claim 6, **characterized in that**
the light guide unit includes an image acquisition unit (108) which acquires an image, and a signal transmission member (114) which transmits an image signal acquired by the image acquisition unit,
the image acquisition unit is disposed close to the first light emitting member or the second light emitting member,
the connection unit includes an image signal terminal (116) which is connectable to an image processing unit (112) to process the image signal and by which the image signal transmitted by the signal transmission member is transmittable to the image processing unit, and
the light source system further includes an image signal transmission route to transmit the image signal acquired by the image acquisition unit to the image processing unit via the image acquisition unit, the signal transmission member and the image signal terminal.
